# EUROPÄISCHE PATENTSCHRIFT

(11) **EP 0 226 093 B1**
(45) Veröffentlichungstag und Bekanntmachung des Hinweises auf die Patenterteilung: **16.06.1993**
(21) Anmeldenummer: 86116509.0
(22) Anmeldetag: 27.11.1986
(51) Int. Cl.: C07D 501/36

(54) **Verfahren zur Herstellung von Cefodizim**
Process for the preparation of cefodizime
Procédé pour la préparation de céfodizime

(30) Priorität: 03.12.1985 DE 3542644
(43) Veröffentlichungstag der Anmeldung: 24.06.1987
(73) Patentinhaber: HOECHST AKTIENGESELLSCHAFT, 65926 Frankfurt am Main (DE)
(72) Erfinder: Jaenicke, Ottmar, Dr., D-6233 Kelkheim/Taunus (DE); Wagner, Hans, Dr., D-6246 Glashütten/Taunus (DE); Worm, Manfred, Dr., D-6200 Wiesbaden (DE)

(56) Entgegenhaltungen:
- EP-A- 0 023 453
- EP-A- 0 124 889
- EP-A- 0 125 576
- FR-A- 2 385 722

## Beschreibung

Cefodizim (HR 221) der Formel 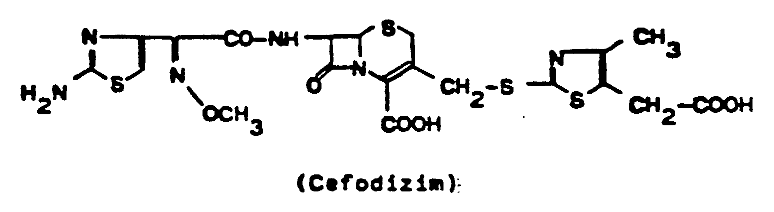
ist ein Antibiotikum, dessen Herstellung und gute antibiotische Wirksamkeit literaturbekannt ist. Es kann erhalten werden durch Umsetzung von an der Carboxylgruppe aktivierter ATS der Formel 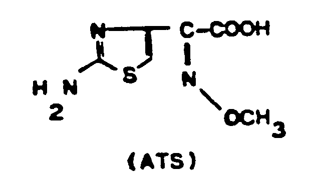
mit TACS der Formel 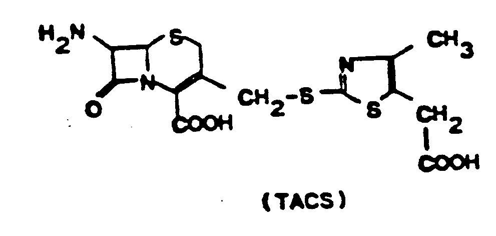
Ein besonders geeignetes Verfahren zur Herstellung von Cephalosporinen wird in der EP-A-23 453 beschrieben. Wendet man es zur Herstellung von Cefodizim an, so entsteht jedoch neben dem gewünschten Cefodizim ein Nebenprodukt in der Größenordnung von etwa 5 bis 15 %. Der Gehalt an Nebenprodukt kann durch verschiedene aufeinanderfolgende Reinigungsschritte vermindert, aber nicht vollständig aus dem Cefodizim entfernt werden. Außerdem sind diese Reinigungsverfahren (z.B. Chromatographie, Umkristallisation) kostenintensiv, da sie umständlich sind und die Gesamtausbeute erheblich mindern. In dem in EP-A-23 453 beschriebenen Verfahren wird kein Silylierungsreagenz verwendet.

In FR 2 385 722 werden 7-E2-(Aminothiazol-4-yl)-2-methoxyimino-acetamido-cephem-Derivate sowie Verfahren zu deren Herstellung beschrieben, wobei als letzter Schritt in 3-Stellung durch die Mercaptothiazolgruppe substituiert wird.

Aus EP-A-23 453 ist ein Verfahren zur Herstellung von 7-[(2-Aryl)-2-hydroxyiminoacetamido]cephem-Derivaten bekannt, bei dem kein Silylierungsreagenz verwendet wird.

Es wurde deshalb weiterhin eine Reihe von Versuchen unternommen, die Reaktionsparameter zu ändern, mit dem Ziel, die Bildung des Nebenproduktes zu unterdrücken, wie z.B. Variation der molaren Verhältnisse, der Temperatur, der Reaktionszeit, des Lösungsmittels, der gegebenenfalls zuzusetzenden Base, des Aktivierungsagens oder durch andere Zusätze. Keiner dieser Versuche brachte einen Erfolg. Die Bildung des erwähnten Nebenproduktes konnte nicht vermindert werden.

Bei der Untersuchung weiterer Zusätze wurde nun überraschenderweise gefunden, daß durch Zusatz eines Silylierungsmittels zu der TACS die Bildung des Nebenproduktes nahezu vollständig verhindert wird.

Die Erfindung betrifft somit ein Verfahren zur Herstellung von Cefodizim der Formel 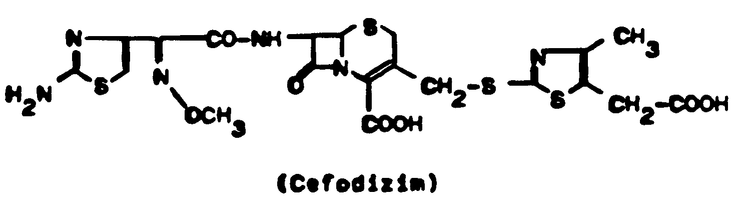
das dadurch gekennzeichnet ist, daß man
(a) zunächst in einem organischen Lösungsmittel und gegebenenfalls in Gegenwart einer Base eine Verbindung der Formel II (ATS) 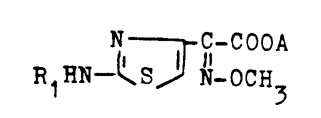 worin R₁ Wasserstoff oder eine Aminoschutzgruppe und A ein Wasserstoffatom oder ein Äquivalent eines Alkali- oder Erdalkalimetalls, Ammonium oder einer organischen Stickstoffbase darstellt, mit einer Verbindung der Formel III

   R-SO₂-Hal

   worin R für Methyl, Methylphenyl oder Phenyl, steht und Hal ein Halogenatom darstellt, umsetzt,
(b) in einem organischen Lösungsmittel und gegebenenfalls in Gegenwart einer Base eine Verbindung der Formel I (TACS) 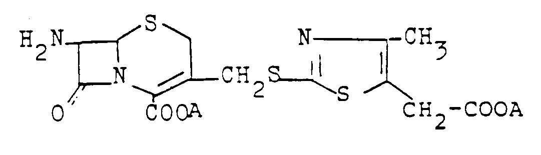 in der A die obige Bedeutung hat, mit einem Silylierungsmittel umsetzt und
(c) die beiden nach (a) und (b) entstandenden Produkte in ihren Reaktionslösungen zur Umsetzung bringt und im Endprodukt der Formel I eine gegebenenfalls vorhandene Schutzgruppe R₁ abspaltet.

A kann außer für Wasserstoff oder Ammonium auch stehen für ein Äquivalent eines Alkalimetalls, wie z.B. Natrium oder Kalium, eines Erdalkalimetalls, wie z.B. Calcium oder Magnesium oder einer organischen Stickstoffbase, wie z.B. Diethyl-, Trimethyl-, Triethyl-, Methyl-, Propyl-, N,N-Dimethylethanol- oder Ethanol-amin.

Als Schutzgruppen R₁ kommen solche in Betracht, die in der Cephalosporin- oder Peptidchemie als Aminoschutzgruppen beschrieben sind, beispielsweise diejenigen, die in EP-A-23453, S. 3, Zeilen 43 - 63 erwähnt werden. Bevorzugt werden erfindungsgemäß Verbindungen eingesetzt, in denen R₁ für Wasserstoff steht. Durch den nicht obligatorischen Schutz der Aminogruppe können so zwei Reaktionsstufen (Schutz, Abspaltung) eingespart werden, was zu einer noch weiteren Ausbeutesteigerung führt.

Als Verbindungen der Formel III kommen alle in Betracht, die in der Literatur als zur Umsetzung mit einer Carbonsäure geeignet beschrieben werden, wie z.B. Methansulfonylchlorid Phenylsulfonchlorid oder Tosylchlorid, wobei ein aktivierter Carbonsäurerest -COOSO₂R entstehen dürfte. Wegen der guten Reaktionsfähigkeit und leichten Zugänglichkeit werden Verbindungen wie p-Tosylchlorid oder auch Phenylsulfonylchlorid bevorzugt. Bevorzugt sind weiterhin Verbindungen der Formel III, in denen Hal für Chlor steht.

Die Umsetzung zwischen der Verbindung der Formel II und einer Verbindung der Formel III kann in einem wasserfreien organischen Lösungsmittel, wie z.B. Aceton, Ethylacetat, Tetrahydrofuran, Acetonitril, Tetrachlorkohlenstoff, Methylenchlorid, Toluol, Dioxan, Isopropylether, N-Methylpyrrolidon, Dimethylformamid, vorzugsweise jedoch in Dimethylacetamid bei Temperaturen zwischen etwa -30 bis 0°C, vorzugsweise zwischen -10 und -15°C durchgeführt werden.

Wird die Verbindung der Formel II in Form der freien Säure (A= Wasserstoff) eingesetzt, ist es zweckmäßig, eine Base, vorzugsweise eine organische Stickstoffbase, wie beispielsweise Triethylamin, N,N-Dimethylanilin,Tributylamin, N-Methyl-morpholin, Pyridin, Picolin oder auch beispielsweise Natrium- oder Kaliumcarbonat oder -bicarbonat, insbesondere Triethylamin zuzusetzen. Die Zugabe der Base kann entfallen, wenn die Carbonsäure der Formel II in Form eines der obengenannten Salze eingesetzt wird.

Die weitere Umsetzung wird nun so durchgeführt, daß die Verbindung der Formel I (TACS) in einem wasserfreien organischen Lösungsmittel, wie z.B. Methylenchlorid, Dimethylacetamid, Methyl-tert. butyläther, Methylisobutylketon, Butylacetat oder Amylacetat, insbesondere Methylenchlorid oder Methyl-tert.-butyläther, mit etwa 3 Moläquivalenten eines Silylierungsmittels, vorzugsweise mit Trimethylchlorsilan, in Gegenwart einer - bezogen auf das Silylierungsmittel - etwa stöchiometrischen Menge einer organischen Stickstoffbase, insbesondere von Triethylamin, umgesetzt und dann mit der durch Umsetzung mit einer Verbindung der Formel III aktivierten Carbonsäure der Formel II zur Umsetzung gebracht wird.

Anstelle von Trimethylchlorsilan können auch andere Silylierungsmittel , wie z.B. Bis-trimethylsilylacetamid, Dichlordimethylsilan, Trichlormethylsilan oder N,Nʹ-Bis-trimethylsilylharnstoff eingesetzt werden, wobei von diesen weiteren Silylierungsmitteln Bis-trimethylsilylacetamid bevorzugt ist. Ein geringer, die 3 Moläquivalente überschreitender Überschuß des Silylierungsmittels wirkt sich nicht schädlich auf die Umsetzung aus.

Bei Verwendung von Bis-trimethylsilylacetamid oder N,Nʹ-Bis-trimethylsilylharnstoff ist es nicht erforderlich, eine Base bei der Silylierung zuzusetzen, da in diesem Fall keine zu bindende Chlorwasserstoffsäure auftritt.

Die Silylierung der Verbindung der Formel I wird bei Temperaturen zwischen etwa 20 bis 25°C durchgeführt. Man kann sie beispielsweise auch auf etwa 40°C (Siedepunkt von Methylenchlorid) ansteigen lassen.

Das Zusammenbringen der aktivierten ATS mit der silylierten TACS erfolgt zweckmäßigerweise so, daß die Lösung der aktivierten ATS bei Temperaturen zwischen etwa -20 bis 0°C, vorzugsweise zwischen -10 bis -12°C, so langsam zu der Lösung der silylierten TACS hinzugegeben wird, daß die auftretende Reaktionswärme gut abgeführt werden kann, beispielsweise innerhalb einer Zeitspanne von 1/4 bis 2 Stunden.

Nach kurzem Nachrühren wird die Reaktionsmischung dann in an sich bekannter Weise aufgearbeitet. So kann sie beispielsweise in Wasser gegossen werden, dessen pH-Wert durch Zugabe von beispielsweise einer organischen Stickstoffbase, insbesondere von Triethylamin, bei etwa 6,0 - 7,5 gehalten wird. Nach der Phasentrennung kann die organische Phase dann nochmals mit Wasser, in dem Natriumacetat gelöst ist, extrahiert werden. Aus den vereinigten Wasserphasen, die gegebenenfalls noch beispielsweise mit Aktivkohle geklärt werden können, kann dann das Cefodizim als freie Säure durch Zugabe einer Mineralsäure, beispielsweise von Schwefelsäure, durch Einstellung des pH-Wertes auf etwa 2,8 ausgefällt werden.

Falls die Carbonsäure der Formel II mit einer Aminoschutzgruppe R₁ eingesetzt wurde, muß vor der Aufarbeitung noch die Abspaltung dieses Restes in literaturbekannter Weise erfolgen.

Nach Trocknung bei leicht erhöhter Temperatur und Vakuum wird so die Cefodizim-Säure in sehr reiner Form erhalten.

Zusätzlich zu der oben bereits erwähnten, nicht zu erwartenden Reduzierung des Nebenproduktes auf einen weit unter 1 % liegenden Gehalt sei noch darauf hingewiesen, daß z.B. in den Ausführungsbeispielen 8 bis 12 der bereits erwähnten EP-A-23 453 bei der Acylierung Reaktionstemperaturen zwischen -70 bis -72°C angewandt werden. Demgegenüber ist es möglich, die erfindungsgemäße Umsetzung bei Temperaturen von etwa -20 bis 0°C durchzuführen, ohne daß es dabei zu einer Qualitäts- oder Ausbeuteverschlechterung kommt. Diese Tatsache hat für die technische Produktion von Cefodizim erhebliches Interesse, da damit kürzere Belegzeiten und eine geringere Kühlleistung verbunden sind.

### Beispiel 1

### Stufe 1

17,5 kg (87,1 Mol) ATS werden in 52 kg Dimethylacetamid mit 8,95 kg (88,6 Mol) Triethylamin in das Ammoniumsalz überführt bei einer Temperatur von 20 bis 22°C und einer Rührzeit von 30 Minuten.

12,9 kg (68,0 Mol) p-Toluolsulfochlorid werden bei 20 - 25°C innerhalb von 30 Minuten in 13,2 kg Dimethylacetamid gelöst und diese Lösung bei -10°C bis -14°C zu der Suspension des Triethylammoniumsalzes der ATS innerhalb von 30 Minuten zulaufen gelassen und weitere 2,5 Stunden gerührt.

### Stufe 2

23,7 kg (59,0 Mol) TACS werden in 212 kg Methylenchlorid suspendiert, 19,7 kg (181,0 Mol) Trimethylchlorsilan bei 20 - 25°C zulaufen gelassen und 18,2 kg (180,0 Mol) Triethylamin innerhalb von 30 Minuten bei einer Temperatur von 20 - 41°C (Rückflußtemperatur des Methylenchlorids) zugegeben.

Nach Zugabeende wird die Lösung sehr schnell auf -15°C abgekühlt und die nach Stufe 1 erhaltene Lösung der aktivierten ATS innerhalb von 30 Minuten bei -10 bis -12°C zugegeben. Nach einer Rührzeit von weiteren 5 Minuten wird die Reaktionsmischung innerhalb von 5 bis 10 Minuten zu einem Gemisch von 143 l Wasser und 19,3 kg Triethylamin laufen gelassen, wobei der pH-Wert zwischen 6 und 7,5 gehalten wird. Nach der Phasentrennung wird die Methylenchloridphase nochmals mit 70 l Wasser, in dem 2,2 kg Natriumacetat.3 H₂O gelöst sind, extrahiert und aus den vereinigten Wasserphasen nach Klärung mit 1,2 kg Aktivkohle und 1,2 kg Dicalite das Cefodizim in Form der freien Säure durch Zugabe von 18-%iger Schwefelsäure bis zum Erreichen eines pH-Wertes von 2,8 gefällt. Nach Abfiltrieren und Trocknen bei 40°C und 100 Torr erhält man 30,6 kg Cefodizim in einer Reinheit von 96 - 97 Flächen-% gemäß HPLC-Analyse.

### Beispiel 2

### Stufe 1 entspricht den Angaben in Beispiel 1.

### Stufe 2

23,7 kg (59,0 Mol) TACS werden in 212 kg Methylenchlorid suspendiert und mit 36,0 kg (177,0 Mol) Bis-trimethylsilylacetamid versetzt. Bei 20 - 25°C wird 30 Minuten gerührt, die klare Lösung auf -15°C abgekühlt und die Lösung der nach Stufe 1 erhaltenen aktivierten ATS innerhalb von 30 Minuten bei -10 bis -12°C zugegeben. Nach einer Rührzeit von 5 Minuten wird die Reaktionsmischung innerhalb von 5 bis 10 Minuten zu 143 l Wasser laufen gelassen und gleichzeitig der pH-Wert mit Triethylamin zwischen pH 6,0 bis 7,5 gehalten. Nach der Phasentrennung wird die organische Phase nochmals mit 70 kg Wasser, das 2,2 kg Natriumacetat.3H₂O enthält, extrahiert. Die vereinigten, wässrigen Phasen werden mit 1,2 kg Aktivkohle und 1,2 kg Dicalite geklärt und das Produkt durch Zugabe von 18-%iger Schwefelsäure bis zum Erreichen eines pH-Wertes von 2,8 gefällt. Nach Abfiltieren und Trocknen bei 40°C und 100 Torr erhält man 30,0 kg Cefodizim in Form der freien Säure in einer Reinheit von 95 bis 97 Flächen-% gemäß HPLC-Analyse.

## Patentansprüche

1. Verfahren zur Herstellung von Cefodizim der Formel 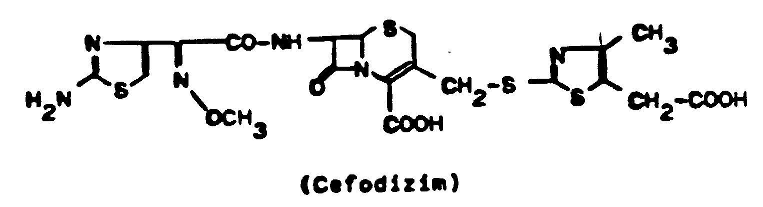 dadurch gekennzeichnet, daß man
(a) zunächst in einem organischen Lösungsmittel und gegebenenfalls in Gegenwart einer Base eine Verbindung der Formel II (ATS) 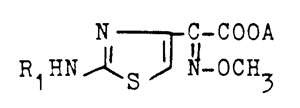 worin R₁ Wasserstoff oder eine Aminoschutzgruppe und A ein Wasserstoffatom oder ein Äquivalent eines Alkali- oder Erdalkalimetalls, Ammonium oder einer organischen Stickstoffbase darstellt, mit einer Verbindung der Formel III
R-SO₂-Hal
worin R für Methyl, Methylphenyl oder Phenyl, steht und Hal ein Halogenatom darstellt, umsetzt,
(b) in einem organischen Lösungsmittel und gegebenenfalls in Gegenwart einer Base eine Verbindung der Formel I (TACS) 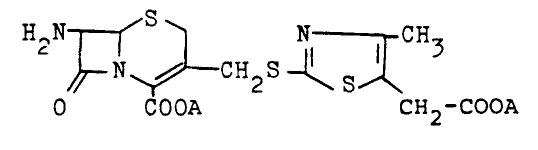 in der A die obige Bedeutung hat, mit einem Silylierungsmittel umsetzt und
(c) die beiden nach (a) und (b) entstandenden Produkte in ihren Reaktionslösungen zur Umsetzung bringt und im Endprodukt der Formel I eine gegebenenfalls vorhandene Schutzgruppe R₁ abspaltet.

2. Verfahren gemäß Anspruch 1, dadurch gekennzeichnet, daß in der Verbindung der Formel III R für p-Tolyl oder Phenyl und Hal für Chlor steht.

3. Verfahren nach Anspruch 2, dadurch gekennzeichnet, daß R für p-Tolyl steht.

4. Verfahren gemäß Ansprüchen 1 bis 3, dadurch gekennzeichnet, daß in der Verbindung der Formel II (ATS) R₁ und A für Wasserstoff stehen.

5. Verfahren nach Ansprüchen 1 bis 5, dadurch gekennzeichnet, daß das in Verfahrensstufe (a) eingesetzte Lösungsmittel Aceton, Dimethylacetamid, Ethylacetat, Tetrahydrofuran, Acetonitril, Tetrachlorkohlenstoff, Methylenchlorid, Toluol, Dioxan, Isopropyläther, n-Methylpyrrolidon oder Dimethylformamid ist.

6. Verfahren nach Anspruch 5, dadurch gekennzeichnet, daß das Lösungsmittel Dimethylacetamid ist.

7. Verfahren nach Ansprüchen 1 bis 6, dadurch gekennzeichnet, daß das Silylierungsmittel Trimethylchlorsilan, Dichlordimethylsilan, Trichlormethylsilan, Bis-trimethylsilylacetamid oder N,N'-Bistrimethylsilylharnstoff ist.

8. Verfahren nach Anspruch 7, dadurch gekennzeichnet, daß das Silylierungsmittel Trimethylchlorsilan ist.

9. Verfahren nach Anspruch 7 und 8, dadurch gekennzeichnet, das 3 Moläquivalente des Silylierungsmittels, bezogen auf die Verbindung der Formel I (TACS), eingesetzt werden.

10. Verfahren gemäß Ansprüchen 1 bis 8, dadurch gekennzeichnet, daß das in Verfahrensstufe (b) eingesetzte Lösungsmittel Methylenchlorid, Dimethylacetamid, Methyl-tert.-butyläther, Methylisobutylketon, Butylacetat oder Amylacetat ist.

11. Verfahren gemäß Anspruch 9, dadurch gekennzeichnet, daß das Lösungsmittel Methylenchlorid oder Methyl-tert.-butyläther ist.

12. Verfahren gemäß Ansprüchen 1 bis 10, dadurch gekennzeichnet, daß die während der einzelnen Verfahrensstufen gegebenenfalls zugegebene Base Triethylamin ist.

13. Verfharen gemäß Ansprüchen 1 bis 11, dadurch gekennzeichnet, daß die Umsetzung der Verfahrensstufe (a) bei Temperaturen zwischen etwa -30 und 0°C durchgeführt wird.

14. Verfahren gemäß Ansprüchen 1 bis 12, dadurch gekennzeichnet, daß die Umsetzung der Verfahrensstufe (b) bei Temperaturen zwischen etwa -20 und 0°C durchgeführt wird.

## Claims

1. A process for the preparation of cefodizime of the formula 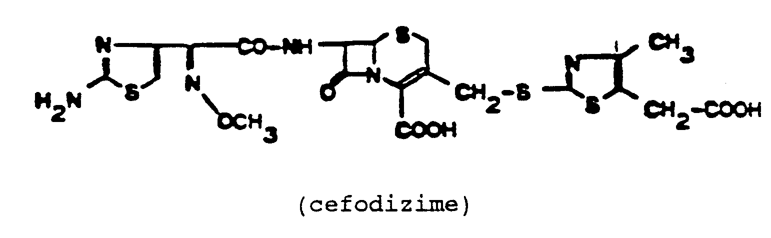 which comprises
(a) first reacting a compound of the formula II (ATS) 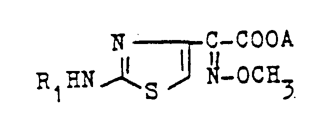 wherein R₁ represents hydrogen or an aminoprotective group and A represents a hydrogen atom or one equivalent of an alkali metal or alkaline earth metal, of ammonium or of an organic nitrogen base, with a compound of the formula III
R-SO₂-Hal
wherein R represents methyl, methylphenyl or phenyl and Hal represents a halogen atom, in an organic solvent and, if appropriate, in the presence of a base,
(b) reacting a compound of the formula I (TACS) 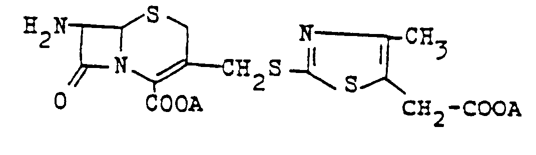 in which A has the above meaning, with a silylating agent in an organic solvent and, if appropriate, in the presence of a base, and
(c) reacting the two products formed in (a) and (b) in their reaction solutions, and eliminating any protective group R₁ present in the end product of the formula I.

2. The process as claimed in claim 1, wherein, in the compound of the formula III, R represents p-tolyl or phenyl and Hal represents chlorine.

3. The process as claimed in claim 2, wherein R represents p-tolyl.

4. The process as claimed in claims 1 to 3, wherein, in the compound of the formula II (ATS), R₁ and A represent hydrogen.

5. The process as claimed in claims 1 to 5, wherein the solvent employed in process stage (a) is acetone, dimethylacetamide, ethyl acetate, tetrahydrofuran, acetonitrile, carbon tetrachloride, methylene chloride, toluene, dioxane, isopropyl ether, n-methylpyrrolidone or dimethylformamide.

6. The process as claimed in claim 5, wherein the solvent is dimethylacetamide.

7. The process as claimed in claims 1 to 6, wherein the silylating agent is trimethylchlorosilane, dichlorodimethylsilane, trichloromethylsilane, bistrimethylsilylacetamide or N,N'-bistrimethylsilylurea.

8. The process as claimed in claim 7, wherein the silylating agent is trimethylchlorosilane.

9. The process as claimed in claims 7 and 8, wherein 3 mol equivalents, based on the compound of the formula I (TACS), of the silylating agent are employed.

10. The process as claimed in claims 1 to 8, wherein the solvent employed in process stage (b) is methylene chloride, dimethylacetamide, methyl tert.-butyl ether, methyl isobutyl ketone, butyl acetate or amyl acetate.

11. The process as claimed in claim 9, wherein the solvent is methylene chloride or methyl tert.-butyl ether.

12. The process as claimed in claims 1 to 10, wherein the base which, if appropriate, is added during the individual process stages is triethylamine.

13. The process as claimed in claims 1 to 11, wherein the reaction of process stage (a) is carried out at temperatures between about -30 and 0°C.

14. The process as claimed in claims 1 to 12, wherein the reaction of process stage (b) is carried out at temperatures between about -20 and 0°C.

## Revendications

1. Procédé de préparation de céfodizime de formule: 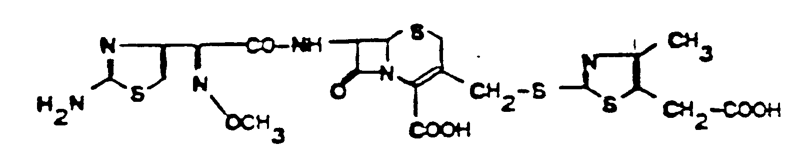 caractérisé en ce que
(a) on fait d'abord réagir, dans un solvant organique et éventuellement en présence d'une base, un composé (ATS) de formule II: 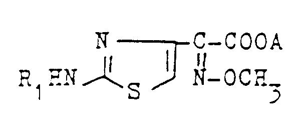 dans laquelle R₁ est un atome d'hydrogène ou un groupe protecteur de la fonction amine, et A représente un atome d'hydrogène ou un équivalent d'un métal alcalin ou alcalinoterreux, d'ammonium ou d'une base azotée organique, avec un composé de formule III:
R-SO₂-Hal ,
dans laquelle R représente méthyle, méthylphényle ou phényle et Hal est un atome d'halogène,
(b) on fait réagir, dans un solvant organique et éventuellement en présence d'une base, un composé (TACS) de formule I: 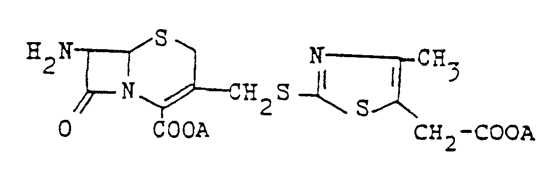 dans laquelle A a la définition indiquée ci-dessus, avec un agent silylant, et
(c) on fait réagir, dans leurs solutions réactionnelles, les deux produits formés selon (a) et (b), et on détache, dans le produit final de formule I, le groupe protecteur R₁ éventuellement présent.

2. Procédé selon la revendication 1, caractérisé en ce que, dans le composé de formule III, R représente p-tosyle ou phényle et Hal est l'atome de chlore.

3. Procédé selon la revendication 2, caractérisé en ce que R est p-tosyle.

4. Procédé selon les revendications 1 à 3, caractérisé en ce que, dans le composé de formule II (ATS), R₁ et A représentent l'atome d'hydrogène.

5. Procédé selon les revendications 1 à 4, caractérisé en ce que le solvant mis en oeuvre dans l'étape opératoire (a) est: acétone, diméthylacétamide, acétate d'éthyle, tétrahydrofurane, acétonitrile, tétrachlorure de carbone, chlorure de méthylène, toluène, dioxane, éther isopropylique, N-méthylpyrrolidone ou diméthylformamide.

6. Procédé selon la revendication 5, caractérisé en ce que le solvant est le diméthylacétamide.

7. Procédé selon les revendications 1 à 6, caractérisé en ce que l'agent silylant est le triméthylchlorosilane, le dichlorodiméthylsilane, le trichlorométhylsilane, le bis-triméthylsilylacétamide ou la N,N'-bis-triméthylsilylurée.

8. Procédé selon la revendication 7, caractérisé en ce que l'agent silylant est le triméthylchlorosilane.

9. Procédé selon les revendications 7 et 8, caractérisé en ce qu'on utilise 3 équivalents molaires d'agent silylant, par rapport au composé (TACS) de formule I.

10. Procédé selon les revendications 1 à 8, caractérisé en ce que le solvant mis en oeuvre dans l'étape opératoire (b) est: chlorure de méthylène, diméthylacétamide, éther de méthyle et de tert-butyle, méthylisobutylcétone, acétate de butyle ou acétate d'amyle.

11. Procédé selon la revendication 9, caractérisé en ce que le solvant est le chlorure de méthylène ou l'éther de méthyle et de tert-butyle.

12. Procédé selon les revendications 1 à 10, caractérisé en ce qu'on ajoute éventuellement la base triéthylamine au cours des diverses étapes opératoires.

13. Procédé selon les revendications 1 à 11, caractérisé en ce que la réaction de l'étape opératoire (a) est réalisée à des températures comprises entre -30 et 0°C.

14. Procédé selon les revendications 1 à 12, caractérisé en ce que la réaction de l'étape opératoire (b) est réalisée à des températures comprises entre -20 et 0°C.
